# FASCICULE DE BREVET EUROPEEN

(11) **EP 1 030 673 B1**
(45) Date de publication et mention de la délivrance du brevet: **28.07.2004**
(21) Numéro de dépôt: 98955662.6
(22) Date de dépôt: 16.11.1998
(51) Int. Cl.: A61K 31/7048

(54) **UTILISATION DES KETOLIDES POUR PREVENIR LES COMPLICATIONS THROMBOTIQUES ARTERIELLES LIEES A L'ARTHEROSCLEROSE**
VERWENDUNG VON KETOLIDEN ZUR VORBEUGUNG VON MIT ATHEROSKLEROSE ZUSAMMENHÄNGENDER THROMBOTISCHER KOMPLIKATIONEN
USE OF KETOLIDES FOR PREVENTING ARTERIAL THROMBOTIC COMPLICATIONS RELATED TO ATHEROSCLEROSIS

(30) Priorité: 17.11.1997 FR 9714358
(43) Date de publication de la demande: 30.08.2000
(73) Titulaire: Aventis Pharma S.A., 92160 Antony (FR)
(72) Inventeur: PETIT, Francis, F-92700 Colombes (FR); VACHERON, Françoise, F-75019 Paris (FR)
(86) Numéro de dépôt international: PCT/FR1998/002436
(87) Numéro de publication internationale: WO 1999/025365

(56) Documents cités:
- EP-A- 0 676 409
- EP-A- 0 680 967
- H. JOHNSSON ET AL.: "Effects of some antibiotics on plateletbfunction in vitro and in vivo." THROMB. RES., vol. 11, no. 2, 1977, pages 237-252, XP002068932
- S. CRONBERG ET AL.: "Investigations on the effect of antimicrobial drugs on platelet aggregation in vitro and ex vivo." FOLIA HAEMATOL., vol. 111, no. 6, 1984, pages 725-734, XP002068933

## Description

La présente invention concerne une nouvelle application thérapeutique des kétolides.

L'invention a pour objet l'utilisation des kétolides et de leurs sels pharmaceutiquement acceptables pour la préparation de compositions pharmaceutiques destinées à prévenir les complications thrombotiques artérielles liées à l'athérosclérose.

On appelle kétolide les dérivés de l'érythromycine dépourvues du cladinose en position 3. Ces produits présentent des propriétés antibiotiques (Antimicrobial Agents and Chemotherapy 1997, Vol. 41, p. 2149 à 2158, ou 1997 vol. 41, p. 454 à 459 ou Lettre de l'infectiologue 1997, vol. 12, p..46 à 54.

Les kétolides sont également décrits par exemple dans les brevets européens 0487411, 596802, 606024, 614905, 676409, 680967, 799833 et la demande de brevet international WO 9825942.

Parmi les kétolides préférés de l'invention, on peut citer les composés de formule (I) : 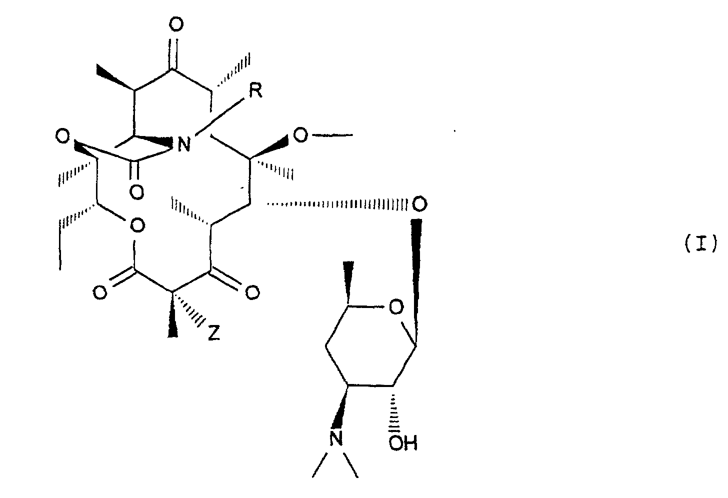 dans laquelle R représente un radical

(CH₂)ₘOₙ(X)YAr

dans lequel m représente le nombre 0 ou 1,
n représente le nombre 0 ou 1,
X représente un radical (NH)ₐ, CH₂ ou SO₂ avec a représentant le nombre 0 ou 1,
Y représente un radical (CH₂)_{b}-(CH=CH)_{c}-(CH₂)_{d} avec c = 0 ou 1, b + c + d ≤ 8,
Z représente un atome d'hydrogène ou d'halogène,
Ar représente un radical aryle ou hétéroaryle éventuellement substitué.

Le radical aryle peut être un radical phényle ou naphtyle.

Le radical hétéroaryle substitué ou non peut être le radical thiényle, furyle, pyrolyle, thiazolyle, oxazolyle, imidazolyle, par exemple le radical 4-(3-pyridinyl) 1H-imidazolyle, thiadiazolyle, pyrazolyle ou isopyrazolyle, un radical pyridyle, pyrimidyle, pyridazinyle ou pyrazinyle, ou encore un radical indolyle benzofurannyle, benzothiazyle ou quinoléinyle.

Ces radicaux aryles peuvent comporter un ou plusieurs groupements choisis dans le groupe constitué par les radicaux hydroxyle, les atomes d'halogène, les radicaux NO₂, les radicaux C≡N, les radicaux alkyle, alkényle ou alkynyle, 0-alkyle, 0-alkényle ou 0-alkynyle, S-alkyle, S-alkényle ou S-alkynyle et N-alkyle, N-alkényle ou N-alkynyle, renfermant jusqu'à 12 atomes de carbone éventuellement substitués par un ou plusieurs atomes d'halogène, le radical 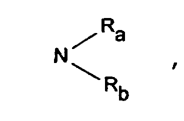 Ra et Rb identiques ou différents, représentant un atome d'hydrogène ou un radical alkyle renfermant jusqu'à 12 atomes de carbone, le radical 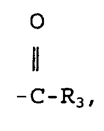 R₃ représentant un radical alkyle renfermant jusqu'à 12 atomes de carbone, ou un radical aryle ou hétéroaryle éventuellement substitué, les radicaux aryle, O-aryle ou S-aryle carboxycliques ou aryle, O-aryle ou S-aryle hétérocycliques à 5 ou 6 chaînons comportant un ou plusieurs hétéro-atomes, éventuellement substitués par un ou plusieurs des substituants mentionnés ci-dessous.
Comme hétéroaryle préféré, on peut citer entre autres 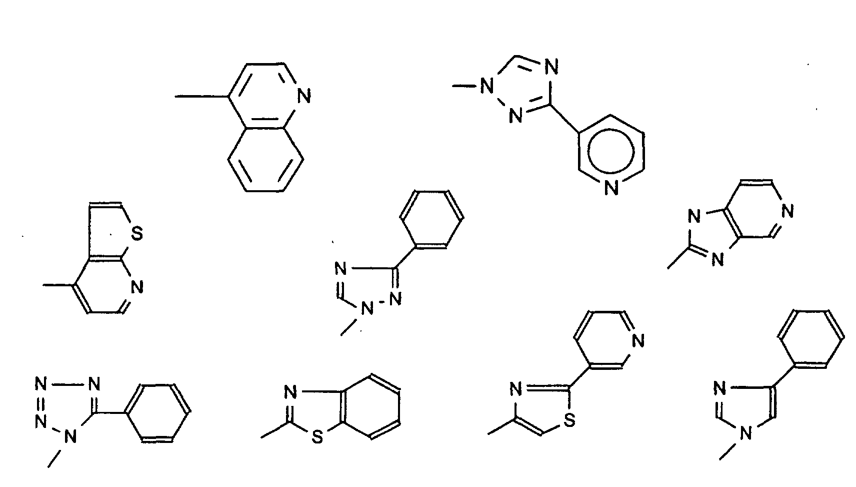 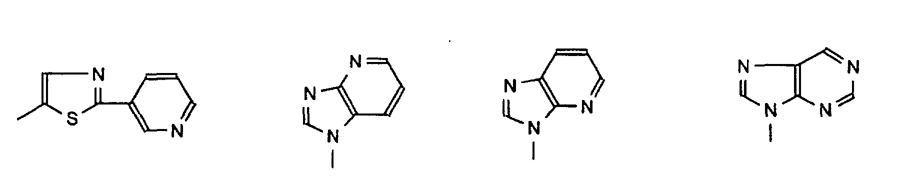 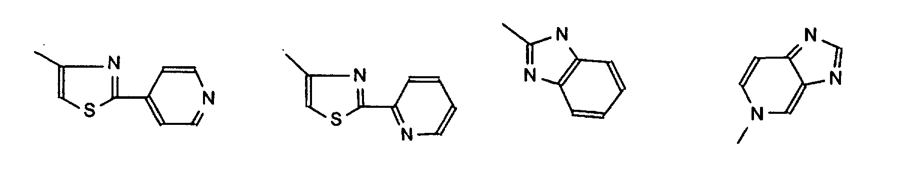 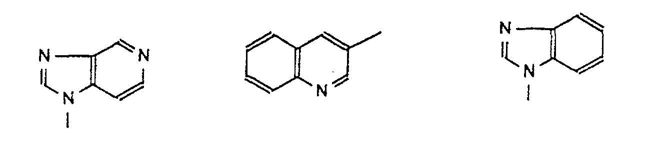 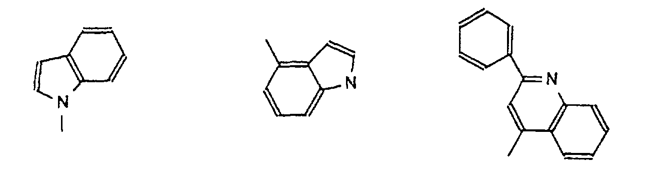 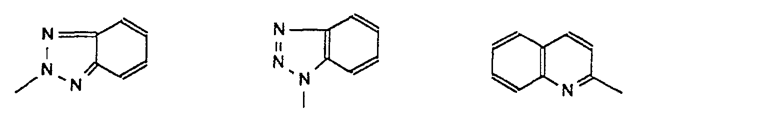 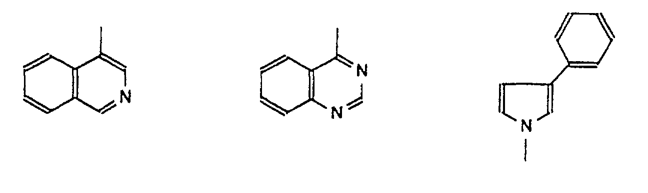 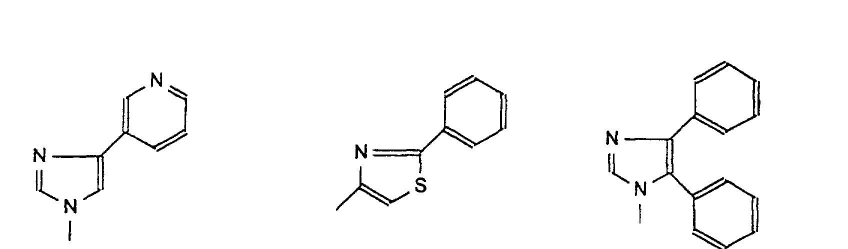 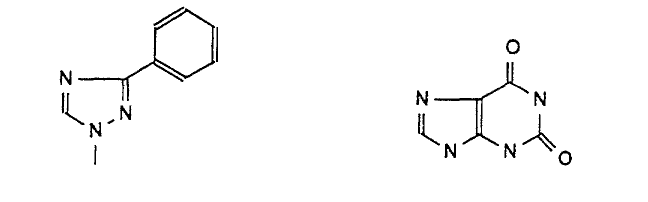 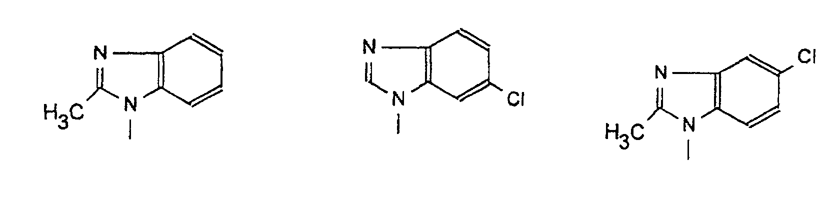 et les radicaux hétérocycliques envisagés dans les demandes de brevets européens 487411, 596802, 676409 et 680967. Ces radicaux hétérocycliques préférés peuvent être substitués par un ou plusieurs groupements fonctionnels.

Halogène représente de préférence un atome de fluor, de chlore ou de brome.

Parmi les sels d'addition avec les acides, on peut citer les sels formés avec les acides acétique, propionique, trifluoroacétique, malique, tartrique, méthanesulfonique, benzènesulfonique, p-toluènesulfonique, et spécialement les acides stéarique, éthylsuccinique ou laurylsulfonique.

Le radical aryle est de préférence un radical arylhétérocyclique. Parmi les kétolides préférés, on peut citer les composés dans lesquels Ar représente un radical

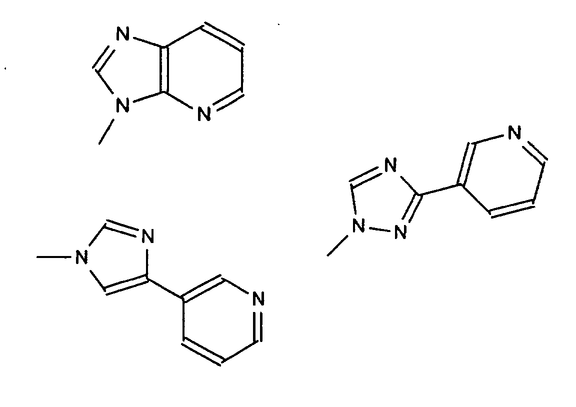

Parmi les composés préférés de l'invention, on peut citer les composés de formule (I) dont les noms suivent : la 11,12-didéoxy-3-de [(2,6-didéoxy-3-C-méthyl-3-O-méthyl-α-L-ribo-hexopyranosyl) oxy] 6-O-méthyl-3-oxo 12,11([oxycarbonyl [[[2-[4-(3-pyridinyl) 1H-imidazol-1-yl] éthoxy] méthyl] imino]] érythromycine (composé P)décrit dans la demande de brevet WO 9825942 à l'exemple 2 ou bien la 11,12-didéoxy-3-de((2,6-didéoxy-3-C-méthyl-3-O-méthyl-α-L-ribohexopyranosyl) oxy) 6-O-méthyl-3-oxo 12,11-(oxycarbonyl ((4-(3-(3-pyridinyl) 1H-1,2,4-triazol-1-yl) butyl) imino) érythromycine (composé P₁) décrit dans le brevet EP 680967 à l'exemple 35, ou bien la 11,12-didéoxy-3-de((2,6-didéoxy-3-C-méthyl-3-O-méthyl-alpha-L-ribohexopyranosyl)oxy)-2-fluoro-6-O-méthyl-3-oxo-12,11-(oxycarbonyl((4-(4-(3-pyridinyl)-1H-imidazol-1-yl) butyl)imino))-érythromycine(isomère A)(composé P₂) décrit dans le brevet EP 799833 à l'exemple 3, ou encore la 11,12-didéoxy-3-de((2,6-didéoxy-3-C-méthyl-3-O-méthyl-alpha-L-ribohexopyranosyl)oxy)-6-O-méthyl-3-oxo-12,11-(oxycarbonyl((4-(4-(3-pyridinyl)-1H-imidazol-1-yl) butyl)imino))-érythromycine (composé P₃) décrit dans le brevet EP 680967 à l'exemple 34.

Parmi les kétolides particulièrement intéressants, on peut citer les produits des brevets européens 676409, 680967 et 799833.

Les kétolides présentent une activité antiagrégante plaquettaire et antithrombotique comme le montrent les résultats obtenus dans la partie expérimentale exposée ci-après.

L'invention a donc pour objet les compositions pharmaceutiques destinées à la prévention des complications artérielles comme les accidents vasculaires cérébraux, l'infarctus du myocarde et l'angor instable consécutifs à l'athérosclérose.

L'agent infectieux Clamydia pneumoniae paraît jouer un rôle dans le développement de l'athérosclérose chez l'homme.

Les kétolides sont actifs contre Clamydia pneumoniae.

De ce fait, les propriétés antiinfectieuses contre Clamydia pneumoniae jointes à leur activité antiagrégante plaquettaire permettent leur utilisation pour s'opposer au développement de l'athérosclérose et des complications thrombotiques.

L'invention a également pour objet les compositions pharmaceutiques renfermant un kétolide défini précédemment destinées à prévenir les complications thrombotiques artérielles liées à l'athérosclérose.

Ces compositions peuvent être administrées par voie bucale, rectale, parentérale ou par voie locale en application topique sur la peau et les muqueuses mais la voie d'administration est la voie buccale.

Elles peuvent être solides ou liquides et se présenter sous les formes pharmaceutiques couramment utilisées en médecine humaine, comme par exemple les comprimés simples ou dragéifiés, les gélules, les granulés, les suppositoires, les préparations injectables, les pommades, les crèmes, les gels; elles sont préparées selon les méthodes usuelles. Le ou les principes actifs peuvent y être incorporés à des excipients habituellement employés dans ces compositions pharmaceutiques, telles que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants et les conservateurs.

Ces compositions peuvent également se présenter sous forme d'une poudre destinées à être dissoute extemporanément dans un véhicule approprié par exemple de l'eau stérile apyrogène.

La dose administrée est variable selon l'infection traitée, le sujet en cause, la voie d'administration et le produit considéré. Elle peut être par exemple comprise entre 50 et 600 mg par jour par voie orale chez l'adulte pour le produit P, P₁, P₂ ou P₃.

### ETUDE PHARMACOLOGIOUE

### AGREGATION PLAQUETTAIRE IN VITRO.

### Principe

L'agrégation plaquettaire est mesurée selon la méthode turbidimétrique inspirée de Born [1] en détectant la transmission optique à travers un plasma riche en plaquettes (PRP) auquel un agent agrégant a été ajouté. Lorsque les plaquettes agrègent, le plasma s'éclaircit et la transmission optique augmente.

### Préparation du plasma riche en plaquettes

Le sang est prélevé (3 tubes par lapin) par ponction cardiaque chez un lapin, dans des tubes contenant du citrate de sodium. Pour obtenir le plasma riche en plaquettes (PRP), les tubes sont centrifugés à 160 g pendant 10 minutes. Les surnageants sont recueillis (PRP) et le culot est remis à centrifuger à 2000 g pendant 15 minutes pour obtenir le plasma pauvre en plaquettes (PPP). Par dilution avec le PPP, le PRP est ajusté à une concentration de 300 000 plaquettes par mm³ ± 10%. Le comptage est effectué à l'aide du compteur Coulter ZM.

### Aorrégation

Des tubes contenant 320 µl de PRP sont mis à incuber à +37°C pendant 30 minutes dans les puits de préincubation.

L'agrégomètre est étalonné avec le PPP pour une transmission optique de 100% correspondant à une agrégation complète et avec le PRP issu du même lapin pour une transmission optique de 0% correspondant à l'absence d'agrégation.

Le produit à étudier P est ajouté sous un volume de 40 µl. Après 2 minutes d'incubation, l'agent agrégant (ADP 10 µM, Arachidonate de sodium 0,2mM ou collagène 20 %g/ml) est ajouté sous un volume de 40 µl. L'agrégation commence immédiatement et peut être visualisée sur l'imprimante.

Sur le tracé obtenu, la hauteur de la courbe d'agrégation est mesurée en cm à partir de la ligne de base avant addition de l'agent agrégant puis traduite en mVolts (=1/DO) en utilisant la formule 10 mV = 2,5 cm.
[1] - Born G.V.R., Agregation of blood platelets by adenosine diphosphate and its reversal, Nature, 1962, 194, 927.

Les résultats obtenus sont les suivants :

### Effet du produit P sur l'agrégation plaquettaire in vitro - Comparaison avec l'aspirine.

| % d'inbition de l'agrégation induite par l'acide arachidonique ⁺ | | |
|---|---|---|
| Concentrations | Produit P * | Aspirine ** |
| 10⁻⁷M | 7 | - |
| 10⁻⁶M | 42 | 8 |
| 10⁻⁵M | 73 | 13 |
| 5x10⁻⁵M | - | 85 |
| 10⁻⁴M | 90 | 100 |

| | | |
|---|---|---|
| ⁺ Les plaquettes de lapin sont mises en présence du produit à différentes concentrations puis l'acide arachidonique est ajouté à la concentration de 0,2 mM. | | |
| * n = 2 lapins | | |
| ** n = 4 lapins sauf pour la concentration 5x10⁻⁵M où n = 2. | | |

Les produits préférés P₁, P₂ et P₃ cités ci-dessus présentent aussi une bonne activité sur ce test d'agrégation plaquettaire in vitro.

## Revendications

1. Utilisation des kétolides et de leurs sels pharmaceutiquement acceptables pour la préparation de compositions pharmaceutiques destinées à prévenir les complications thrombotiques artérielles liées à l'athérosclérose.

2. Utilisation selon la revendication 1, **caractérisée en ce que** le kétolide répond à la formule (I) : 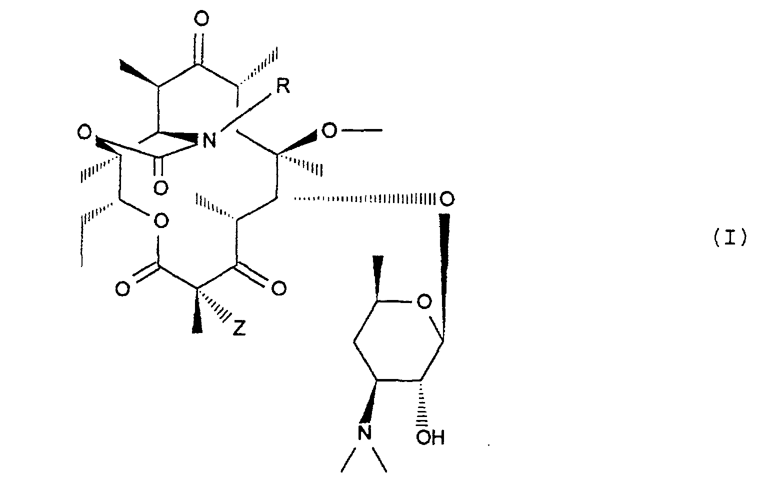 dans laquelle R représente un radical
(CH₂)ₘOₙ(X)YAr
dans lequel m représente le nombre 0 ou 1,
n représente le nombre 0 ou 1,
X représente un radical (NH)ₐ, CH₂ ou SO₂ avec a représentant le nombre 0 ou 1,
Y représente un radical (CH₂)_{b}-(CH=CH)_{c}-(CH₂)_{d} avec c = 0 ou 1, b + c + d s 8,
Z représente un atome d'hydrogène ou d'halogène,
Ar représente un radical aryle ou hétéroaryle éventuellement substitué.

3. Utilisation selon la revendication 1 ou 2, **caractérisé en ce que** le kétolide est la 11,12-didéoxy-3-de [(2,6-didéoxy-3-C-méthyl-3-O-méthyl-α-L-ribo-hexopyranosyl) oxy] 6-o-méthyl-3-oxo 12,11([oxycarbonyl [[[2-[4-(3-pyridinyl) 1H-imidazol-1-yl] éthoxy] méthyl] imino]] érythromycine.

4. Utilisation selon l'une quelconque des revendications 1 à 3, **caractérisée en ce que** le kétolide est la 11,12-didéoxy-3-de((2,6-didéoxy-3-C-méthyl-3-O-méthyl-α-L-ribohexopyranosyl) oxy) 6-O-méthyl-3-oxo-12,11-(oxycarbonyl ((4-(3-(3-pyridinyl) 1H-1,2,4-triazol-1-yl) butyl) imino) érythromycine.

5. Utilisation selon l'une quelconque des revendications 1 à 3, **caractérisée en ce que** kétolide est la 11,12-didéoxy-3-de((2,6-didéoxy-3-C-méthyl-3-O-méthyl-alpha-L-ribohexopyranosyl)oxy)-2-fluoro-6-O-méthyl-3-oxo-12,11-(oxycarbonyl((4-(4-(3-pyridinyl)-1H-imidazol-1-yl) butyl)imino))-érythromycine(isomère A)

6. Utilisation selon l'une quelconque des revendications 1 à 3, **caractérisée en ce que** kétolide est la 11,12-didéoxy-3-de((2,6-didéoxy-3-C-méthyl-3-O-méthyl-alpha-L-ribohexopyranosyl)oxy)-6-O-méthyl-3-oxo-12,11-(oxycarbonyl((4-(4-(3-pyridinyl)-1H-imidazol-1-yl) butyl)imino))-érythromycine

7. Utilisation selon l'une quelconque des revendications 1 à 6, **caractérisée en ce que** kétolide est administré par voie orale à une dose comprise entre 50 et 600 mg par jour.

## Claims

1. Use of ketolides and their pharmaceutically acceptable salts for the preparation of pharmaceutical compositions intended for preventing arterial thrombolytic complications linked to atherosclerosis.

2. Use according to Claim 1, **characterized in that** the ketolide corresponds to formula (I): 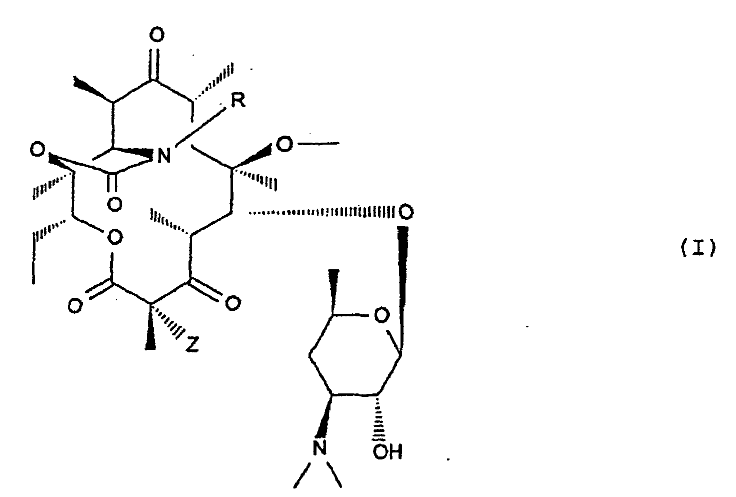 in which R represents a radical
(CH₂)ₘOₙ(X)YAr
in which m represents the number 0 or 1,
n represents the number 0 or 1,
X represents a radical (NH)ₐ, CH₂ or SO₂ with a representing the number 0 or 1,
Y represents a radical (CH₂)_{b}-(CH=CH)_{c}-(CH₂)_{d} with c = 0 or 1, b + c + d ≤ 8,
Z represents a hydrogen or halogen atom,
Ar represents an optionally substituted aryl or heteroaryl radical.

3. Use according to Claim 1 or 2, **characterized in that** the ketolide is 11,12-dideoxy-3-de[(2,6-dideoxy-3-C-methyl-3-O-methyl-α-L-ribohexopyranosyl)oxy]-6-O-methyl-3-oxo-12,11-([oxycarbonyl[[[2-[4-(3-pyridinyl)-1H-imidazol-1-yl]ethoxy]methyl]imino]]erythromycin.

4. Use according to any one of Claims 1 to 3, **characterized in that** the ketolide is 11,12-dideoxy-3-de((2,6-dideoxy-3-C-methyl-3-O-methyl-α-L-ribohexapyranosyl)oxy)-6-O-methyl-3-oxo-12,11-(oxycarbonyl((4-(3-(3-pyridinyl)-1H-1,2,4-triazol-1-yl)butyl)imino)erythromycin.

5. Use according to any one of Claims 1 to 3, **characterized in that** the ketolide is 11,12-dideoxy-3-de((2,6-dideoxy-3-C-methyl-3-O-methyl-alpha-L-ribohexopyranosyl)oxy)-2-fluoro-6-O-methyl-3-oxo-12,11-(oxycarbonyl((4-(4-(3-pyridinyl)-1H-imidazol-1-yl)butyl)imino))erythromycin (A isomer).

6. Use according to any one of Claims 1 to 3, **characterized in that** the ketolide is 11,12-dideoxy-3-de((2,6-dideoxy-3-C-methyl-3-O-methyl-alpha-L-ribohexopyranosyl)oxy)-6-O-methyl-3-oxo-12,11-(oxycarbonyl((4-(4-(3-pyridinyl)-1H-imidazol-1-yl)butyl)imino))erythromycin.

7. Use according to any one of Claims 1 to 6, **characterized in that** the ketolide is administered orally at a dose of between 50 and 600 mg per day.

## Patentansprüche

1. Verwendung von Ketoliden und ihren pharmazeutisch unbedenklichen Salzen zur Herstellung von pharmazeutischen Zusammensetzungen zur Prophylaxe von mit Atherosklerose zusammenhängenden arteriellen thrombotischen Komplikationen.

2. Verwendung nach Anspruch 1, **dadurch gekennzeichnet, dass** das Ketolid der Formel (I) entspricht: 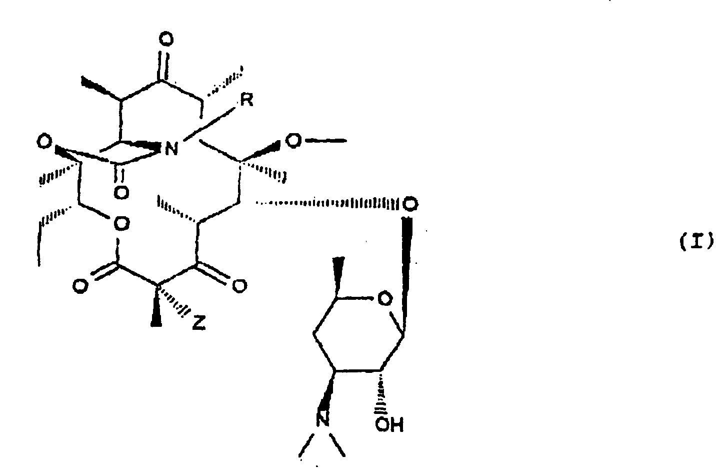 in welcher R für einen Rest
(CH₂)ₘOₙ(X)YAr
steht, worin m für die Zahl 0 oder 1 steht,
n für die Zahl 0 oder 1 steht,
X für einen Rest (NH)ₐ, CH₂ oder SO₂ steht, wobei a für die Zahl 0 oder 1 steht,
Y für einen Rest (CH₂)_{b}-(CH=CH)_{c}-(CH₂)_{d} steht, wobei c = 0 oder 1, b + c + d ≤ 8,
Z für ein Wasserstoff- oder ein Halogenatom steht und
Ar für einen gegebenenfalls substituierten Aryl- oder Heteroarylrest steht.

3. Verwendung nach Anspruch 1 oder 2, **dadurch gekennzeichnet, dass** es sich bei dem Ketolid um 11,12-Didesoxy-3-des-[(2,6-didesoxy-3-C-methyl-3-O-methyl-α-L-ribohexopyranosyl)oxy]-6-O-methyl-3-oxo-12,11-([oxycarbonyl-[[[2-[4-(3-pyridinyl)-1Himidazol-1-yl]ethoxy]methyl]imino]]exythromycin handelt.

4. Verwendung nach einem der Ansprüche 1 bis 3, **dadurch gekennzeichnet, dass** es sich bei dem Ketolid um 11,12-Didesoxy-3-des-((2,6-didesoxy-3-C-methyl-3-O-methyl-α-L-ribohexopyranosyl)oxy)-6-O-methyl-3-oxo-12,11-(oxycarbonyl-((4-(3-(3-pyridinyl)-1H-1,2,4-triazol-1-yl)butyl)imino)-erythromycin handelt.

5. Verwendung nach einem der Ansprüche 1 bis 3, **dadurch gekennzeichnet, dass** es sich bei dem Ketolid um 11,12-Didesoxy-3-des-((2,6-didesoxy-3-C-methyl-3-O-methyl-alpha-L-ribohexopyranosyl)-oxy)-2-fluor-6-O-methyl-3-oxo-12,11-(oxycarbonyl-((4-(4-(3-pyridinyl)-1H-imidazol-1-yl)butyl)-imino))-erythromycin (Isomer A) handelt.

6. Verwendung nach einem der Ansprüche 1 bis 3, **dadurch gekennzeichnet, dass** es sich bei dem Ketolid um 11,12-Didesoxy-3-des-((2,6-didesoxy-3-C-methyl-3-O-methyl-alpha-L-ribohexopyranosyl)-oxy)-6-O-methyl-3-oxo-12,11-(oxycarbonyl-((4-(4-(3-pyridinyl)-1H-imidazol-1-yl)butyl)imino))-erythromycin handelt.

7. Verwendung nach einem der Ansprüche 1 bis 6, **dadurch gekennzeichnet, dass** das Ketolid auf oralem Weg in einer Dosis zwischen 50 und 600 mg pro Tag verabreicht wird.
